# EUROPEAN PATENT APPLICATION

(11) **EP 4 597 092 A1**
(43) Date of publication of application: **06.08.2025**
(21) Application number: 23872514.7
(22) Date of filing: 28.09.2023
(51) Int. Cl.: G01N 27/30

(54) **ELECTRODE, AND ELECTROCHEMICAL MEASURING SYSTEM**

(30) Priority: 30.09.2022 JP 2022157553
(71) Applicant: Nitto Denko Corporation, Ibaraki-shi Osaka 567-8680 (JP)
(72) Inventor: SUETSUGI, Tomokazu, Ibaraki-shi, Osaka 567-8680 (JP); HAISHI, Motoki, Ibaraki-shi, Osaka 567-8680 (JP)
(74) Representative: Gill Jennings & Every LLP
(86) International application number: PCT/JP2023/035374
(87) International publication number: WO 2024/071292

(57) **Abstract**

An electrode (1) includes a substrate film (2) and a conductive carbon layer (4) in order toward one side in a thickness direction. A ratio (N/C) of nitrogen to carbon on a one-side surface of the conductive carbon layer in the thickness direction is measured by using X-ray photoelectron spectroscopy, and the ratio is 0.001 or more.

## Description

### TECHNICAL FIELD

The present invention relates to an electrode and an electrochemical measurement system.

### BACKGROUND ART

Conventionally, there has been known a carbon electrode including a substrate and a conductive carbon layer in order in the thickness direction (for example, see Patent Document 1 below).

### Citation List

### Patent Document

Patent Document 1: Japanese Unexamined Patent Publication No. 2021-056205

### SUMMARY OF THE INVENTION

### PROBLEM TO BE SOLVED BY THE INVENTION

The electrode is required to have excellent sensitivity to the object to be measured. The electrode described in Patent Document 1 may not sufficiently satisfy the above-described requirement.

The present invention provides an electrode and an electrochemical measurement system that have excellent sensitivity to the object to be measured.

### MEANS FOR SOLVING THE PROBLEM

The present invention [1] includes an electrode including: a substrate film; and a conductive carbon layer in order toward one side in a thickness direction, wherein a ratio (N/C) of nitrogen to carbon on a one-side surface of the conductive carbon layer in the thickness direction is measured by using X-ray photoelectron spectroscopy, and is 0.001 or more.

The present invention [2] includes the electrode described in the above-described [1], wherein a ratio (N/C) of nitrogen to carbon on an other-side surface of the conductive carbon layer in the thickness direction is measured by using X-ray photoelectron spectroscopy, and is lower than the ratio (N/C) of nitrogen to carbon on the one-side surface.

The present invention [3] includes the electrode described in the above-described [1] or [2] further including: a metal underlying layer disposed between the substrate film and the conductive carbon layer, wherein the metal underlying layer is in contact with a one-side surface of the substrate film in the thickness direction and an other-side surface of the conductive carbon layer in the thickness direction.

The present invention [4] includes the electrode described in any one of the above-described [1] to [3], being an electrode for an electrochemical measurement.

The present invention [5] includes an electrochemical measurement system including: the electrode described in any one of the above-described [1] to [4].

### EFFECTS OF THE INVENTION

According to the present invention, the ratio (N/C) of nitrogen to carbon on the one-side surface of the conductive carbon layer in the thickness direction is 0.001 or more. Therefore, the electrode and the electrochemical measurement system including the electrode have excellent sensitivity to the object to be measured.

### BRIEF DESCRIPTION OF THE DRAWINGS

FIG. 1 is a cross-sectional view of one embodiment of the electrode of the present invention.
FIG. 2 is a schematic view of one embodiment of the electrochemical measurement system of the present invention.

### DESCRIPTION OF THE EMBODIMENT

### 1. Electrode

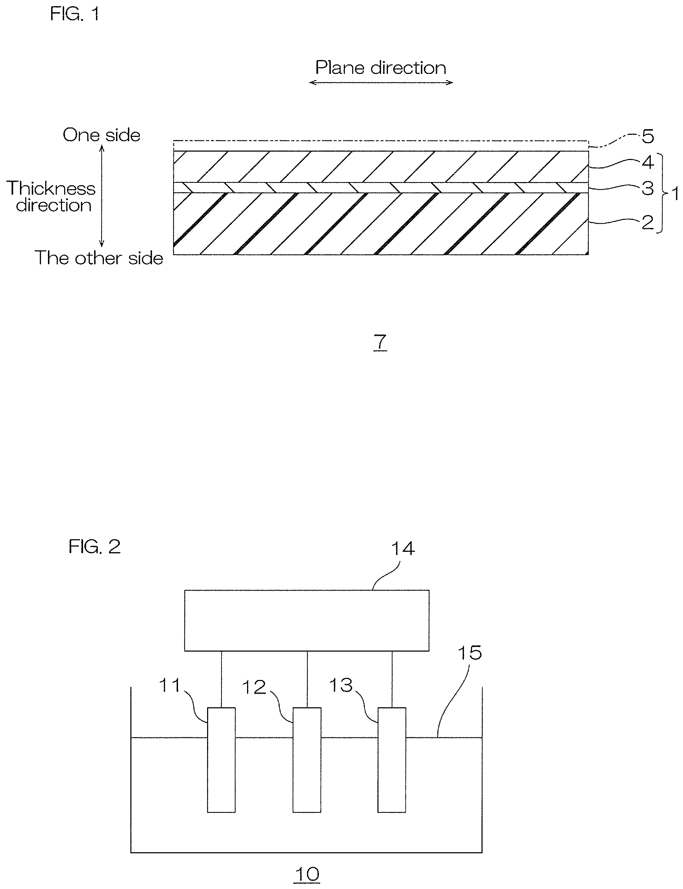

Referring to FIG. 1, one embodiment of the electrode of the present invention is described.

An electrode 1 has a thickness. The electrode 1 extends in a plane direction. The plane direction is orthogonal to a thickness direction. The electrode 1 has a film shape. The electrode 1 has a thickness of, for example, 2 µm or more, preferably 10 µm or more, and is, for example, 1000 µm or less, preferably 500 µm or less. In the present embodiment, the electrode 1 includes a substrate film 2, a metal underlying layer 3, and a conductive carbon layer 4 in order toward one side in the thickness direction. Preferably, the electrode 1 includes only the substrate film 2, the metal underlying layer 3, and the conductive carbon layer 4.

### 1.1 Substrate Film 2

The substrate film 2 extends in the plane direction. Examples of the material of the substrate film 2 include resins and ceramics. The material of the substrate film 2 is preferably a resin from the viewpoint of ensuring flexibility. The resin has flexibility. Examples of the resin include polyester resin, acetate resin, polyether sulfone resin, polycarbonate resin, polyamide resin, polyimide resin, polyolefin resin, (meth)acrylic resin, polyvinyl chloride resin, polyvinylidene chloride resin, polystyrene resin, polyvinyl alcohol resin, polyarylate resin, and polyphenylene sulfide resin. The resins can be used alone or in combination of two or more. As the resin, preferably a polyester resin is used, and preferably polyethylene terephthalate is used. The substrate film 2 has a thickness of, for example, 1.9 µm or more, preferably 9 µm or more, and, for example, 999 µm or less, preferably 499 µm or less.

### 1.2 Metal Underlying Layer 3

The metal underlying layer 3 is disposed between the substrate film 2 and the conductive carbon layer 4. The metal underlying layer 3 is disposed on a one-side surface of the substrate film 2 in the thickness direction. The metal underlying layer 3 is in contact with the one-side surface of the substrate film 2 in the thickness direction. The metal underlying layer 3 extends in the plane direction.

The metal underlying layer 3 assists the conductivity of the conductive carbon layer 4 in the electrode 1. Examples of the material of the metal underlying layer 3 include titanium, niobium, tantalum, chromium, molybdenum, and tungsten. The materials of the metal underlying layer 3 can be used alone or in combination. As the material of the metal underlying layer 3, from the viewpoint of the stability of the electrode 1, preferably titanium and niobium are used, and more preferably niobium is used. The metal underlying layer 3 has a thickness of, for example, 3 nm or more, preferably 6 nm or more, and, for example, 100 nm or less, preferably 50 nm or less.

### 1.3 Conductive Carbon Layer 4

The conductive carbon layer 4 is disposed on a one-side surface of the metal underlying layer 3 in the thickness direction. The conductive carbon layer 4 is in contact with the one-side surface of the metal underlying layer 3 in the thickness direction. In other words, the metal underlying layer 3 is in contact with the other-side surface of the conductive carbon layer 4 in the thickness direction. The conductive carbon layer 4 extends in the plane direction.

The ratio (N/C) of nitrogen to carbon on a one-side surface of the conductive carbon layer 4 in the thickness direction is 0.001 or more.

When the ratio (N/C) of nitrogen to carbon on the one-side surface of the conductive carbon layer 4 in the thickness direction is less than 0.001, the sensitivity of the electrode 1 is insufficient.

The ratio (N/C) of nitrogen to carbon on the one-side surface of the conductive carbon layer 4 in the thickness direction is preferably 0.005 or more, more preferably 0.009 or more, even more preferably 0.010 or more, particularly preferably 0.015 or more, most preferably 0.020 or more, and even 0.025 or more and even 0.030 or more are preferable. When the ratio (N/C) of nitrogen to carbon on the one-side surface of the conductive carbon layer 4 in the thickness direction is the above-described lower limit or more, the sensitivity of the electrode 1 can further be improved.

The upper limit of the ratio (N/C) of nitrogen to carbon on the one-side surface of the conductive carbon layer 4 in the thickness direction is not limited. The upper limit of the ratio (N/C) of nitrogen to carbon on the one-side surface of the conductive carbon layer 4 in the thickness direction is, for example, 0.5. When the ratio (N/C) of nitrogen to carbon on the one-side surface of the conductive carbon layer 4 in the thickness direction is the above-described upper limit or less, the structure as the carbon layer can be maintained.

In the conductive carbon layer 4, the ratio (N/C) of nitrogen to carbon on the other-side surface is, for example, lower than the ratio (N/C) of nitrogen to carbon on the one-side surface. That is, in the conductive carbon layer 4, the ratio (N/C) of nitrogen to carbon on the one-side surface is higher than the ratio (N/C) of nitrogen to carbon on the other-side surface. In the conductive carbon layer 4, when the ratio (N/C) of nitrogen to carbon on the other-side surface is lower than the ratio (N/C) of nitrogen to carbon on the one-side surface, the sensitivity of the electrode 1 can further be improved.

The ratio (N/C) of nitrogen to carbon on the other-side surface of the conductive carbon layer 4 is, for example, less than 0.001, preferably 0.0005 or less, more preferably 0.0001 or less, and particularly preferably 0.000.

The method of setting the ratios (N/C) of carbon to nitrogen on the one-side surface and the other-side surface of the conductive carbon layer 4 in the thickness direction within the above-described ranges is not limited. Examples of the method of setting the ratio (N/C) of nitrogen to carbon on the one-side surface of the conductive carbon layer 4 in the thickness direction within the above-described range include, for example, a method of nitriding the one-side surface of the conductive carbon layer 4 in the thickness direction, etching, surface modification, and cleaning. In the present embodiment, preferably, a method of nitriding the one-side surface of the conductive carbon layer 4 in the thickness direction is used. The method of nitriding the one-side surface is described later.

The ratio (N/C) of nitrogen to carbon on the one-side surface of the conductive carbon layer 4 in the thickness direction is measured using X-ray photoelectron spectroscopy. The conditions for X-ray photoelectron spectroscopy are described in the Examples below.

The conductive carbon layer 4 may include, for example, an sp² bond and an sp³ bond. When the conductive carbon layer 4 includes an sp² bond and an sp³ bond, the conductive carbon layer 4 has a graphite structure and a diamond structure. In this manner, the sensitivity of the electrode 1 can be improved, and excellent strength is also achieved. The ratio of the number of sp³ bonded atoms to the sum of the number of sp³ bonded atoms and the number of sp² bonded atoms is, for example, 0.1 or more and 0.9 or less.

The conductive carbon layer 4 has a thickness of, for example, 0.1 nm or more, preferably 1 nm or more, and 100 nm or less, preferably 50 nm or less.

### 1.4 Method of Producing Electrode 1

Next, the method of producing the electrode 1 is described.

In this method, first, a substrate film 2 is prepared.

Next, in this method, a metal underlying layer 3 is formed on a one-side surface of the substrate film 2 in the thickness direction. In the present embodiment, the metal underlying layer 3 is formed by using sputtering. In the present embodiment, magnetron sputtering is preferably used. As the material for sputtering, a material of the metal underlying layer 3 described above is used.

The device used in the sputtering can be driven in advance. In this manner, the inside of the device can be ready for sputtering. The above-described driving is carried out before sputtering, and thus referred to as "pre-sputtering". The conditions for the pre-sputtering are the same as those for the sputtering in the formation of the metal underlying layer 3.

Next, in this method, a conductive carbon layer 4 is formed on a one-side surface of the metal underlying layer 3 in the thickness direction. In the present embodiment, the conductive carbon layer 4 is formed by using sputtering. In the present embodiment, magnetron sputtering is preferably used. Sintered carbon is used as the material for sputtering.

The above-described conductive carbon layer 4 does not yet have the above-described ratio (N/C) of nitrogen to carbon.

Next, in the method in the present embodiment, a one-side surface of the conductive carbon layer 4 in the thickness direction is, for example, subjected to a nitriding treatment. The nitriding treatment is not limited. Examples of the nitriding treatment include a nitrogen plasma treatment, a reactive sputtering using nitrogen gas, and a surface modification other than a nitrogen plasma treatment and a reactive sputtering using nitrogen gas. As the nitriding treatment, preferably a nitrogen plasma treatment is used.

Examples of the nitrogen plasma treatment include a nitrogen plasma treatment under atmospheric pressure, a nitrogen plasma treatment under low pressure, high-frequency plasma under low pressure, and microwave plasma under low pressure.

The atmospheric pressure is a pressure that is the atmosphere that is not intentionally reduced during plasma generation, and is, for example, 0.05 MPa or more and 0.15 MPa or less, and specifically, is about 0.1M Pa.

In the nitrogen plasma treatment, the one-side surface of the conductive carbon layer 4 is exposed to the generated plasma in nitrogen atmosphere.

In the nitrogen plasma treatment, a plasma generator (not shown) is used. The plasma generator includes, for example, a stage, a plasma generation unit, a gas supply device, and an application unit, although not shown. On the stage, the conductive carbon layer 4 can be mounted. The plasma generation unit is movable relative to the stage. The plasma generation unit includes a nozzle. The gas supply device is capable of supplying nitrogen into the plasma generation unit. The application unit is disposed in the plasma generation unit. The device electrode is capable of applying a voltage to the nitrogen in the plasma generation unit.

A laminate of the substrate film 2, the metal underlying layer 3, and the conductive carbon layer 4 is placed on the stage. While nitrogen is supplied from the gas supply device into the plasma generation unit, a voltage is applied to the nitrogen in the plasma generation unit by using the device electrode to generate plasma from the plasma generation unit. The plasma generator is moved relative to the stage while the one-side surface of the conductive carbon layer 4 is irradiated with the plasma.

In this manner, an electrode 1 including the substrate film 2, the metal underlying layer 3, and the conductive carbon layer 4 whose one-side surface is nitrided is manufactured.

### 1.5 Application

The application of the electrode 1 is not limited. The electrode 1 can preferably be used as an electrode for electrochemical measurement used for carrying out an electrochemical measurement method, specifically, as a working electrode (working pole) used for carrying out cyclic voltammetry (CV).

Examples of the object (object to be measured) of the electrochemical measurement include ferrocene, phenanthroline dione, methylene blue, 1-Methoxy PMS, NADH, p-nitroaniline, p-aminophenol, ethanol, lactic acid, β-hydroxybutyric acid, glucose, and potassium ferrocyanide. The object of the electrochemical measurement is preferably glucose.

### 1.6 Electrochemical Measurement System

Referring to FIG. 2, one embodiment of the electrochemical measurement system of the present invention is described.

The electrochemical measurement system 10 includes a working electrode 11, a reference electrode 12, a counter electrode 13, a potentiostat 14, and an ammeter (not shown).

The working electrode 11 includes the electrode 1 described above. In this embodiment, preferably, the working electrode 11 is an enzyme electrode 7. Specifically, the enzyme electrode 7 includes the above-described electrode 1 and an enzyme layer 5. In other words, the electrochemical measurement system 10 includes the electrode 1 described above. The enzyme layer 5 is disposed on the one-side surface of the conductive carbon layer 4 in the thickness direction. The enzyme layer 5 is in contact with the one-side surface of the conductive carbon layer 4 in the thickness direction. The enzyme layer 5 contains, for example, an enzyme, an immobilizing agent, and a buffer solution. Examples of oxidase include glucose dehydrogenase. Examples of the immobilizing agent include, for example, albumin and glutaraldehyde. Examples of the buffer solution include a phosphate compound.

Examples of the reference electrode 12 include a silver/silver chloride electrode, a saturated calomel electrode, and a standard hydrogen electrode.

Examples of the counter electrode 13 include a platinum electrode, a gold electrode, and a nickel electrode.

The above-described working electrode 11, reference electrode 12, and counter electrode 13 can be immersed in a target solution 15. The target solution 15 includes the above-described object to be measured, a mediator, a buffer solution, and an electrolyte. Examples of the mediator include potassium ferrocyanide. Examples of the buffer solution include a phosphate compound. Examples of the electrolyte include potassium chloride.

### 2. Operations and Effects

According to the electrode 1, the ratio (N/C) of nitrogen to carbon on the one-side surface of the conductive carbon layer 4 in the thickness direction is high, i.e., 0.001 or more. Therefore, the electrode 1 has excellent sensitivity to the object be measured. In particular, the electrode 1 has excellent sensitivity to glucose.

As a mechanism of the above-described operation, for example, the following [1] and/or [2] are/is assumed.
[1] The one-side surface of the conductive carbon layer 4 in the thickness direction tends to be cationic by being nitrided. On the other hand, when the object to be measured coexists with potassium ferrocyanide as a mediator in the electrolytic solution, the mediator ionizes to form an anion ([Fe(CN) ₆) ⁴⁻) in the electrolytic solution. Then, the cationic one-side surface and the above-described anion are close to each other based on the electrostatic interaction. In this manner, the sensitivity of the electrode 1 to the object to be measured is improved.
[2] On the one-side surface of the conductive carbon layer 4 in the thickness direction, a portion of the carbon array is substituted by nitrogen, so that negative carriers (charged particles) are easily generated. Then, with the generation of negative carriers, the electron transport efficiency increases on the one-side surface of the conductive carbon layer 4, and the electron transfer efficiency with potassium ferrocyanide increases. Therefore, the sensitivity of the electrode 1 to the object to be measured is improved.

### 3. Modified Examples

In the modified examples, the same members as in one embodiment are given the same numerical references, and the detailed descriptions thereof are omitted. Further, the modified examples can have the same operations and effects as those of one embodiment unless especially described otherwise. Furthermore, one embodiment and the modified examples can appropriately be combined.

Although not shown, the electrode 1 may not include a metal underlying layer 3.

Although not shown, the electrode 1 may include two metal underlying layers 3 and two conductive carbon layers 4. Specifically, the electrode 1 includes a conductive carbon layer 4, a metal underlying layer 3, a substrate film 2, a metal underlying layer 3, and a conductive carbon layer 4 in order toward one side in the thickness direction.

### Examples

With reference to Examples and Comparative Examples below, the present invention is more specifically described. The present invention is not limited to Examples and Comparative Examples in any way. The specific numeral values used in the description below, such as blending ratios (content ratios), physical property values, and parameters, can be replaced with the corresponding blending ratios (content ratios), physical property values, and parameters in the above-described "DESCRIPTION OF THE EMBODIMENT", including the upper limit values (numeral values defined with "or less" or "less than") or the lower limit values (numeral values defined with "or more" or "more than").

### Example 1

A substrate film 2 made of polyethylene terephthalate and having a thickness of 188 µm was prepared.

Next, a metal underlying layer 3 made of titanium was formed on the one-side surface of the substrate film 2 in the thickness direction by using magnetron sputtering. The conditions for magnetron sputtering were as follows.

Target material: titanium
Target Power: 3.3 W/cm²
Sputtering gas: argon
Sputtering chamber pressure: 0.2 Pa

The thickness of the metal underlying layer 3 was 8 nm.

Next, a conductive carbon layer 4 was formed on the one-side surface of the metal underlying layer 3 in the thickness direction by magnetron sputtering. The conditions for magnetron sputtering were as follows.

Target material: sintered carbon
Target Power: 3.3 W/cm²
Sputtering gas: argon
Sputtering chamber pressure: 0.2 Pa

The thickness of the conductive carbon layer 4 was 10 nm.

Thereafter, the one-side surface of the conductive carbon layer 4 in the thickness direction was subjected to a nitrogen-plasma treatment at atmospheric pressure (0.1 MPa). The nitrogen plasma was a low-temperature plasma. The conditions of the nitrogen plasma treatment are described below.

Atmosphere gas: nitrogen
Movement rate of the plasma generation unit with respect to the conductive carbon layer 4: 500mm/minute
Plasma gas flow rate: 60 SCCM

In this manner, an electrode 1 including the substrate film 2, the metal underlying layer 3, and the conductive carbon layer 4 whose one-side surface was subjected to a nitrogen plasma treatment was produced.

### Examples 2 to 4

An electrode 1 was produced in the same manner as in Example 1. However, the movement rate of the plasma generation unit with respect to the conductive carbon layer 4 was changed as described in Table 1.

### Example 5

A substrate film 2 made of polyethylene terephthalate and having a thickness of 188 µm was prepared.

Next, a metal underlying layer 3 made of niobium was formed on the one-side surface of the substrate film 2 in the thickness direction by using magnetron sputtering. The conditions for magnetron sputtering were as follows.

Target material: niobium
Target Power: 4.1 W/cm²
Sputtering gas: argon
Sputtering chamber pressure: 0.2 Pa

The thickness of the metal underlying layer 3 was 23 nm.

Next, a conductive carbon layer 4 was formed on the one-side surface of the metal underlying layer 3 in the thickness direction by magnetron sputtering. The conditions for magnetron sputtering were as follows.

Target material: sintered carbon
Target Power: 3.3 W/cm²
Sputtering gas: argon
Sputtering chamber pressure: 0.2 Pa

The thickness of the conductive carbon layer 4 was 5 nm.

Thereafter, the one-side surface of the conductive carbon layer 4 in the thickness direction was treated. Specifically, the one-side surface of the conductive carbon layers 4 in the thickness direction was subjected to a nitrogen plasma treatment at atmospheric pressure (0.1 MPa). The nitrogen plasma was low-temperature plasma. The conditions of the nitrogen plasma treatment are described below.

Atmosphere gas: nitrogen
Movement rate of the nozzle with respect to the conductive carbon layer 4: 400 mm/minute
Plasma gas flow rate: 60 SCCM

In this manner, an electrode 1 including the substrate film 2, the metal underlying layer 3, and the conductive carbon layer 4 whose one-side surface was subjected to a nitrogen plasma treatment was produced.

### Example 6

An electrode 1 was produced in the same manner as in Example 5. However, the movement rate of the nozzle with respect to the conductive carbon layer 4 was changed as described in Table 1.

### Comparative Example 1

An electrode 1 was produced in the same manner as in Example 1. However, the one-side surface of the conductive carbon layer 4 was not subjected to a nitrogen plasma treatment under atmospheric pressure.

### Comparative Example 2

An electrode 1 was produced in the same manner as in Example 1. However, instead of the nitrogen plasma treatment, an oxygen plasma treatment was carried out.

### [Evaluations]

The following items were evaluated with respect to the electrode 1 of each example and each comparative example. The results are shown in Table 1.

### <Ratios (N/C) of Nitrogen to Carbon on One-side Surface and Other-side Surface of Conductive Carbon Layer 4 in Thickness Direction>

The electrode 1 was cut into a size of about a 1-cm square and fixed to a sample stage. A one-side surface of the conductive carbon layer 4 in the thickness direction was evaluated by X-ray photoelectron spectroscopy. Specifically, the one-side surface of the conductive carbon layer 4 in the thickness direction was subjected to wide-scan measurement to carry out a qualitative analysis. In addition, the C element and N element were subjected to narrow scan measurement to calculate the element ratio (atomic %) and thereby calculating the ratio of nitrogen to carbon as an N/C ratio.

The N/C ratio was calculated for the nitrogen-to-carbon ratio at the thickness (depth) of 10 nm (corresponding to the other-side surface) while etching was being carried out with an Ar ion gun. The thickness was calculated as a thickness (depth) in terms of SiO₂.

### <X-ray Photoelectron Spectrometer and X-ray Photoelectron Spectroscopy Measurement>

X-ray photoelectron spectrometer: ULVAC FIE Quantera SXM
X-ray source: monochrome Al Kα
X Ray setting: 100 µmφ [15 kV, 25 W]
Photoelectron extraction angle: 45 degrees with respect to the sample surface
Charge neutralization: use of neutralization gun and Ar ion-gun (neutralization mode) in combination
Accelerating Voltage of Ar ion gun: 1 kV
Raster size of Ar ion gun: 1 mm×1 mm
Etching Rate of Ar ion gun: 4nm/min in terms of SiO₂

In each of the electrodes 1 of Comparative Example 1 and Comparative Example 2, an N element was not detected.

The lower limit on the detection of the ratio (N/C) of nitrogen to carbon in the above-described measurement was 0.0001.

### [Production of Enzyme Electrode 7]

First, 0.8mg of glucose dehydrogenase, 1.5 µL of a 4wt% bovine serum albumin aqueous solution, 1.2 µL of a 1% glutaraldehyde aqueous solution, and 0.3 µL of a 0.05M potassium phosphate buffer solution (pH6.5) were mixed to prepare an enzyme solution.

Next, an insulating tape having a 2mm-diameter hole was attached to a one-side surface of the electrode 1 in the thickness direction, thereby producing an electrode 1 having the one-side surface of a known exposed area was produced. Subsequently, the above-described enzyme solution was added dropwise to the electrode 1, and then the electrode 1 was stored in a refrigerator at 3°C overnight or more. In this manner, an enzyme electrode (enzyme-modified carbon electrode) 7 including the electrode 1 and an enzyme layer 5 was produced.

### [Measurement of Currents in Response to Glucose]

A target solution 15 was prepared. Specifically, a 100mM potassium ferrocyanide solution, an electrolyte solution obtained by adding KCl to a 0.05M phosphate buffer solution (pH6.5) so that the electrolyte solution was 1M, and a 1000mg/dL glucose solution were mixed according to the formulations shown in Table 2 to prepare glucose solutions in concentrations of 0mg/dL, 100mg/dL, 200mg/dL, 400mg/dL, and 600mg/dL, respectively.

Next, the enzyme electrode (enzyme-modified carbon electrode) 7 was used as a working electrode 11, and as shown in FIG. 2, the working electrode 11 was connected together with a reference electrode 12 (Ag/AgCl) and a counter electrode 13 (Pt) to a potentiostat 14 (IVIUM Technologies, pocketSTAT) to produce an electrochemical measurement system 10 including them. Next, 1 mL of the glucose solution of each concentration was then developed on the enzyme-modified carbon electrode for one minute. Thereafter, for the working electrode 11 of each of the electrochemical measurement systems 10, cyclic voltammetry (CV) measurement was carried out in a potential sweep range of -0.2 to 0.8 V at a scan rate of 0.1 V/sec. From the results of CV measurement, the value of the current value of 600mg/dl with respect to the current value of the glucose concentration of 0mg/dl in 0.3V was defined as the signal-to-background ratio. The S/N ratios were evaluated based on the following criteria.

A: The S/N ratio was 50 or more. The sensitivity to glucose was significantly excellent.
B: The S/N ratio was 35 or more and less than 50. The sensitivity to glucose was excellent.
C: The S/N ratio was 30 or more and less than 35. The sensitivity to glucose was slightly excellent.
D: The S/N ratio was less than 30. The sensitivity to glucose was insufficient.

### Table 1

**Table 1**

| Example-Comparative Example | Metal underlying layer | Nitriding treatment | Movement rate of plasma generation unit with respect to one-side surface of conductive carbon layer [mm/min] | Ratio (N/C) of nitrogen to carbon on conductive carbon layer | | Sensitivity of electrode |
|---|---|---|---|---|---|---|
| | | | | One surface | Depth of 10 nm from one surface : the other-side surface | |
| Example 1 | Titanium | Nitrogen plasma | 500 | 0.031 | 0.000^{*2} | A |
| Example 2 | Titanium | Nitrogen plasma | 750 | 0.019 | 0.000^{*2} | B |
| Example 3 | Titanium | Nitrogen plasma | 2,000 | 0.013 | 0.000^{*2} | C |
| Example 4 | Titanium | Nitrogen plasma | 3,000 | 0.009 | 0.000^{*2} | C |
| Example 5 | Niobium | Nitrogen plasma | 400 | 0.050 | 0.000^{*2} | A |
| Example 6 | Niobium | Nitrogen plasma | 600 | 0.024 | 0.000^{*2} | A |
| Comparative Example1 | Titanium | -^{*1} | - | 0.000^{*2} | 0.000^{*2} | D |
| Comparative Example2 | Titanium | (Oxygen plasma) | 500 | 0.000^{*2} | 0.000^{*2} | D |

| | | | | | | |
|---|---|---|---|---|---|---|
| *1 : Untreated *2 : Less than 0.0001 | | | | | | |

### Table 2

**Table 2**

| Glucose concentration in solution [mg/dL] | Parts by mass for formulation (µL) | | |
|---|---|---|---|
| | Potassium ferrocyanide | 1000mg/dL glucose solution | Potassium phosphate buffer solution + KCl |
| 0 | 50 | 0 | 950 |
| 100 | 50 | 100 | 850 |
| 200 | 50 | 200 | 750 |
| 400 | 50 | 400 | 550 |
| 600 | 50 | 600 | 350 |

While the illustrative embodiments of the present invention are provided in the above description, such is for illustrative purpose only and it is not to be construed as limiting in any manner. Modification and variation of the present invention that will be obvious to those skilled in the art is to be covered by the following claims.

### Industrial Applicability

The electrode is used for electrochemical measurement systems.

### Description of Reference Numerals

- 1: Electrode
- 2: Substrate film
- 3: Metal underlying layer
- 4: Conductive carbon layer
- 7: Enzyme electrode
- 10: Electrochemical measurement system
- 11: Working electrode

## Claims

1. An electrode comprising: a substrate film; and a conductive carbon layer in order toward one side in a thickness direction, wherein a ratio (N/C) of nitrogen to carbon on a one-side surface of the conductive carbon layer in the thickness direction is measured by using X-ray photoelectron spectroscopy, and is 0.001 or more.

2. The electrode according to claim 1, wherein a ratio (N/C) of nitrogen to carbon on an other-side surface of the conductive carbon layer in the thickness direction is measured by using X-ray photoelectron spectroscopy, and is lower than the ratio (N/C) of nitrogen to carbon on the one-side surface.

3. The electrode according to claim 1 or 2, further comprising:
a metal underlying layer disposed between the substrate film and the conductive carbon layer,
wherein the metal underlying layer is in contact with a one-side surface of the substrate film in the thickness direction and an other-side surface of the conductive carbon layer in the thickness direction.

4. The electrode according to claim 3, being an electrode for an electrochemical measurement.

5. An electrochemical measurement system comprising: the electrode according to claim 4.
